# EUROPÄISCHE PATENTANMELDUNG

(11) **EP 2 908 384 A1**
(43) Veröffentlichungstag der Anmeldung: **19.08.2015**
(21) Anmeldenummer: 15152268.7
(22) Anmeldetag: 23.01.2015
(51) Int. Cl.: H01R 13/115, H01R 12/71, H01R 11/32, H01R 13/11, H01R 12/75

(54) **Hausgerätevorrichtung**

(30) Priorität: 14.02.2014 ES 201430200
(71) Anmelder: BSH Hausgeräte GmbH, 81739 München (DE)
(72) Erfinder: Aranda Vazquez, Sandra, 50018 Zaragoza (ES); Diez Esteban, Cristina, 50017 Zaragoza (ES); Hernandez Blasco, Pablo Jesus, 50410 Cuarte de Huerva (Zaragoza) (ES); Martin Gomez, Damaso, 20012 Zaragoza (ES); Moya Albertin, Maria Elena, 50002 Zaragoza (ES); Sanchez Garcia, Eva Maria, 50013 Zaragoza (ES)

(57) **Zusammenfassung**

Um eine gattungsgemäße Vorrichtung mit verbesserten Eigenschaften hinsichtlich geringer Kosten und/oder einer hohen Flexibilität bereitzustellen, wird eine Hausgerätevorrichtung (10), insbesondere eine Kochfeldvorrichtung, vorgeschlagen mit zumindest einem ersten Steckverbinder (12), der zumindest ein Paar von ersten Kontaktelementen (14, 16) aufweist, und mit zumindest einem zweiten Steckverbinder (18), der zumindest ein Paar von zweiten Kontaktelementen (20, 22) aufweist, das in wenigstens einem montierten Zustand gemeinsam mit dem zumindest einen Paar von ersten Kontaktelementen (14, 16) zwei zueinander parallele Verbindungen (24, 26) ausbildet, wobei zumindest eines der zweiten Kontaktelemente (20, 22) einen Kabelschuh aufweist.

## Beschreibung

Die Erfindung betrifft eine Hausgerätevorrichtung nach dem Patentanspruch 1.

Aus dem Stand der Technik ist bereits eine Hausgerätevorrichtung bekannt, die einen ersten Steckverbinder und einen zweiten Steckverbinder umfasst. Der erste Steckverbinder weist ein einziges erstes Kontaktelement auf und der zweite Steckverbinder weist ein einziges zweites Kontaktelement auf, das als Kabelschuh ausgebildet ist. Durch eine Verbindung zwischen dem ersten Steckverbinder und dem zweiten Steckverbinder kann ein elektrischer Strom mit einer Stromstärke von maximal 20 A fließen, beispielsweise zur Versorgung wenigstens eines Heizelements. Um einen elektrischen Strom mit einer höheren Stromstärke leiten zu können, wird im Stand der Technik vorgeschlagen, eine stromführende Leitung mit einer Basis zu verschrauben, die ein passendes Gewinde zu einer Aufnahme einer Schraube aufweist. Hierbei muss jedoch das Anziehen der Schraube mit kontrolliertem Drehmoment erfolgen, was zu einer aufwändigeren Montage und damit zu höheren Kosten führt.

Die Aufgabe der Erfindung besteht insbesondere darin, eine gattungsgemäße Vorrichtung mit verbesserten Eigenschaften hinsichtlich geringer Kosten und/oder einer hohen Flexibilität bereitzustellen. Die Aufgabe wird erfindungsgemäß durch die Merkmale des Patentanspruchs 1 gelöst, während vorteilhafte Ausgestaltungen und Weiterbildungen der Erfindung den Unteransprüchen entnommen werden können.

Es wird eine Hausgerätevorrichtung, insbesondere eine Kochfeldvorrichtung, vorgeschlagen mit zumindest einem ersten Steckverbinder, der zumindest ein Paar von ersten Kontaktelementen aufweist und der insbesondere dazu vorgesehen ist, mit zumindest einer Leiterplatte verbunden zu werden und vorteilhaft in zumindest einem Betriebszustand einen elektrischen Strom mit einer Stromstärke von mindestens 25 A zu führen, und mit zumindest einem zweiten Steckverbinder, der zumindest ein Paar von zweiten Kontaktelementen aufweist, das in wenigstens einem montierten Zustand gemeinsam mit dem zumindest einen Paar von ersten Kontaktelementen zwei zueinander parallele insbesondere elektrische Verbindungen ausbildet, wobei zumindest eines der zweiten Kontaktelemente einen Kabelschuh aufweist, insbesondere ausbildet. Unter einer "Hausgerätevorrichtung" soll insbesondere zumindest ein Teil, insbesondere eine Unterbaugruppe, eines Hausgeräts, insbesondere eines Kochfelds, verstanden werden. Insbesondere kann die Hausgerätevorrichtung auch das gesamte Hausgerät, insbesondere das gesamte Kochfeld, umfassen. Unter einem "Steckverbinder" soll insbesondere ein Bauteil verstanden werden, das dazu vorgesehen ist, in wenigstens einem montierten Zustand mit einem weiteren Bauteil, insbesondere mit einem weiteren Steckverbinder, eine werkzeuglos lösbare insbesondere elektrisch leitende Verbindung auszubilden, wobei in dem wenigstens einen montierten Zustand vorzugsweise ein erstes Bauteil der beiden Bauteile in ein zweites Bauteil der beiden Bauteile eingeführt ist und vorteilhaft das zweite Bauteil das erste Bauteil wenigstens teilweise umgreift. Unter einem "Kontaktelement" soll insbesondere ein insbesondere elektrisch leitfähiges Element verstanden werden, das in dem wenigstens einen montierten Zustand wenigstens einen Teil einer werkzeuglos lösbaren insbesondere elektrisch leitenden Verbindung ausbildet. Vorzugsweise wirken ein erstes Kontaktelement und ein zweites Kontaktelement in dem wenigstens einen montierten Zustand zusammen und bilden vorteilhaft die werkzeuglos lösbare insbesondere elektrisch leitende Verbindung aus, wobei das erste Kontaktelement und das zweite Kontaktelement in dem wenigstens einen montierten Zustand insbesondere in punktförmigen, vorzugsweise linienförmigen und vorteilhaft in flächigem Kontakt miteinander angeordnet sind. Unter einem "Kabelschuh" soll insbesondere ein Element verstanden werden, das zumindest einen Aufnahmebereich zu einer Aufnahme wenigstens eines Kontaktelements, insbesondere eines ersten Kontaktelements, aufweist, insbesondere ausbildet, und das wenigstens eine Kontaktelement, insbesondere das erste Kontaktelement, in dem wenigstens einen montierten Zustand wenigstens teilweise umgreift. Insbesondere umgreift das zumindest eine den Kabelschuh aufweisende zweite Kontaktelement das zumindest eine erste Kontaktelement wenigstens in einem ersten Randbereich des zumindest einen ersten Kontaktelements, wobei das zumindest eine zweite Kontaktelement insbesondere mindestens 10 %, vorteilhaft mindestens 15 %, besonders vorteilhaft mindestens 20 % und vorzugsweise mindestens 25 % einer Quererstreckung des zumindest einen ersten Kontaktelements in dem ersten Randbereich umgreift. Unter einer "Quererstreckung" eines Elements soll insbesondere eine Länge einer zweitgrößten Seite eines kleinsten gedachten das Element gerade noch umschließenden Quaders verstanden werden. Unter der Wendung, dass ein erstes Element ein zweites Element "wenigstens teilweise umgreift", soll insbesondere verstanden werden, dass das erste Element ausgehend von einem Mittelpunkt und/oder Schwerpunkt des ersten Elements über einen Winkelbereich von mindestens 180°, insbesondere von mindestens 200°, vorteilhaft von mindestens 220° und vorzugsweise von mindestens 240° das zweite Element umgreift. Das zumindest eine erste Kontaktelement ist vorteilhaft in dem wenigstens einen montierten Zustand in insbesondere flächigem Kontakt mit dem den Kabelschuh aufweisenden zumindest einen zweiten Kontaktelement in dem zumindest einen Aufnahmebereich des zumindest einen den Kabelschuh aufweisenden zweiten Kontaktelements angeordnet. Insbesondere ist das zumindest eine erste Kontaktelement in dem wenigstens einen montierten Zustand über einen Oberflächenanteil von mindestens 50 %, vorteilhaft von mindestens 60 %, besonders vorteilhaft von mindestens 70 % und vorzugsweise von mindestens 80 % einer Seite des zumindest einen ersten Kontaktelements in flächigem Kontakt. Unter einem "Paar" von Elementen soll insbesondere eine Anzahl von genau zwei Elementen verstanden werden. Unter zwei "zueinander parallelen" Verbindungen sollen insbesondere zwei Verbindungen verstanden werden, die in dem wenigstens einen montierten Zustand insbesondere elektrisch parallel geschaltet sind. Insbesondere bilden in dem wenigstens einen montierten Zustand ein erstes Kontaktelement des zumindest einen Paars von ersten Kontaktelementen sowie ein ersten Kontaktelement des zumindest einen Paars von zweiten Kontaktelementen gemeinsam eine erste Verbindung aus und ein zweites Kontaktelement des zumindest einen Paars von ersten Kontaktelementen sowie ein zweites Kontaktelement des zumindest einen Paars von zweiten Kontaktelementen bilden gemeinsam eine zweite Verbindung aus. Unter "vorgesehen" soll insbesondere speziell ausgelegt und/oder ausgestattet verstanden werden. Darunter, dass ein Objekt zu einer bestimmten Funktion vorgesehen ist, soll insbesondere verstanden werden, dass das Objekt diese bestimmte Funktion in zumindest einem Anwendungs- und/oder Betriebszustand erfüllt und/oder ausführt.

Durch die derartige Ausgestaltung können insbesondere geringe Kosten und/oder eine hohe Flexibilität erreicht werden. Besonders vorteilhaft kann auf Befestigungselemente, wie insbesondere Schrauben, verzichtet werden, wodurch insbesondere ein schneller Montagevorgang erreicht werden kann. Darüber hinaus kann eine platzsparende Ausgestaltung erreicht werden, da insbesondere auf eine große Basis zu einer Aufnahme von Schrauben verzichtet werden kann. Vorzugsweise kann eine hohe Verlässlichkeit und/oder Funktionstüchtigkeit erreicht werden, da Fehler in Bezug auf Drehmomente vermieden werden können. Da eine Kontrolle des Drehmoments entfällt, kann insbesondere auf teure Werkzeuge verzichtet werden. Zudem kann eine Verbindung schnell hergestellt und/oder gelöst werden, wodurch im Bedarfsfall flexibel und schnell eine Reparatur erfolgen kann.

Es sind verschiedene, einem Fachmann als sinnvoll erscheinende Ausgestaltungen des zumindest einen zweiten Steckverbinders denkbar. Beispielsweise könnte ein weiteres zweites Kontaktelement des Paars von zweiten Kontaktelementen, insbesondere neben dem den Kabelschuh aufweisenden zweiten Kontaktelement des Paars von zweiten Kontaktelementen, eine Öffnung aufweisen, die zu einer Aufnahme wenigstens eines der ersten Kontaktelemente vorgesehen sein könnte. Ebenfalls denkbar ist, dass das weitere zweite Kontaktelement einen Vorsprung aufweist, der in dem wenigstens einen montierten Zustand in einer Öffnung eines der ersten Kontaktelemente angeordnet sein könnte. Vorzugsweise weisen die zweiten Kontaktelemente des zumindest einen Paars von zweiten Kontaktelementen jedoch jeweils einen Kabelschuh auf, wobei die zweiten Kontaktelemente des zumindest einen Paars von zweiten Kontaktelementen jeweils insbesondere den Kabelschuh ausbilden. Dadurch kann insbesondere eine hohe Flexibilität erreicht werden. Darüber hinaus können Toleranzen vorteilhaft ausgeglichen werden.

Beispielsweise könnten die beiden zueinander parallelen Verbindungen in wenigstens einem Betriebszustand unterschiedliche Stromstärken führen. Vorzugsweise führen jedoch die beiden zueinander parallelen Verbindungen in wenigstens einem Betriebszustand wenigstens im Wesentlichen gleiche Stromstärken. Unter der Wendung, dass die beiden zueinander parallelen Verbindungen in wenigstens einem Betriebszustand "wenigstens im Wesentlichen gleiche" Stromstärken führen, soll insbesondere verstanden werden, dass eine erste Verbindung der beiden zueinander parallelen Verbindungen eine Stromstärke mit einem Wert aufweist, der insbesondere kleiner und/oder gleich ist wie ein Wert einer Stromstärke einer zweiten Verbindung der beiden zueinander parallelen Verbindungen und der mindestens 70 %, insbesondere mindestens 80 %, vorteilhaft mindestens 90 % und vorzugsweise mindestens 95 % des Werts der Stromstärke der zweiten Verbindung beträgt. Dadurch kann insbesondere eine hohe elektrische Effizienz und/oder eine hohe Flexibilität erreicht werden.

Weiterhin wird vorgeschlagen, dass die Hausgerätevorrichtung zumindest eine Leiterplatte umfasst, mit welcher der zumindest eine erste Steckverbinder in dem wenigstens einen montierten Zustand insbesondere fest verbunden ist. Unter einer "Leiterplatte" soll insbesondere ein Element verstanden werden, das dazu vorgehen ist, in dem wenigstens einen montierten Zustand zumindest ein elektronisches Bauteil, insbesondere den zumindest einen ersten Steckverbinder, zu tragen und/oder zu halten, und das insbesondere zu einer mechanischen Befestigung und/oder einer elektrischen Verbindung des zumindest einen elektronischen Bauteils vorgesehen ist. Der zumindest eine erste Steckverbinder ist in dem wenigstens einen montierten Zustand insbesondere an der zumindest einen Leiterplatte fixiert, wobei die zumindest eine Leiterplatte vorteilhaft zumindest eine Leiterbahn aufweist, mit welcher der zumindest eine erste Steckverbinder in dem wenigstens einen montierten Zustand in elektrisch leitendem Kontakt angeordnet ist. Beispielsweise könnte der zumindest eine Steckverbinder mittels einer Rastverbindung, einer Schraubverbindung und/oder einer durch Verriegelung hergestellten Verbindung mit der zumindest einen Leiterplatte verbunden sein. Vorteilhaft ist der zumindest eine Steckverbinder mittels einer Lötverbindung mit der zumindest einen Leiterplatte, insbesondere der zumindest einen Leiterbahn, verbunden. Der zumindest eine Steckverbinder weist insbesondere zumindest ein erstes Eingriffselement auf, das dazu vorgesehen ist, in dem wenigstens einen montierten Zustand durch die zumindest eine Leiterplatte hindurchzugreifen und/oder in die zumindest eine Leiterplatte einzugreifen. Die zumindest eine Leiterplatte weist vorteilhaft zumindest eine Ausnehmung zu einer Aufnahme des zumindest einen ersten Eingriffselements auf. Dadurch kann insbesondere eine hohe Funktionstüchtigkeit erreicht werden. Zudem kann vorteilhaft eine hohe Stromstärke durch den zumindest einen ersten Steckverbinder geführt werden.

Ferner wird vorgeschlagen, dass der zumindest eine erste Steckverbinder zumindest ein erstes Führungselement aufweist, das dazu vorgesehen ist, eine Herstellung einer Verbindung zwischen dem zumindest einen ersten Steckverbinder und dem zumindest einen zweiten Steckverbinder insbesondere in einer Montagerichtung, die vorteilhaft parallel zu einer Längsrichtung des zumindest einen ersten Steckverbinders ausgerichtet ist, zu erleichtern. Das zumindest eine erste Führungselement ist insbesondere zusätzlich dazu vorgesehen, eine Herstellung einer Verbindung zwischen dem zumindest einen ersten Steckverbinder und dem zumindest einen zweiten Steckverbinder insbesondere in einer wenigstens im Wesentlichen senkrecht zu der Montagerichtung ausgerichteten Querrichtung zu erleichtern. Die Querrichtung ist vorteilhaft wenigstens im Wesentlichen parallel zu einer Querrichtung wenigstens eines der ersten Kontaktelemente ausgerichtet und verläuft vorteilhaft parallel zu einer Haupterstreckungsebene des wenigstens einen ersten Kontaktelements. Hierbei ist die Querrichtung des wenigstens einen der ersten Kontaktelemente insbesondere parallel zu der Quererstreckung des wenigstens einen der ersten Kontaktelemente ausgerichtet. Unter einer "Haupterstreckungsebene" eines Objekts soll insbesondere eine Ebene verstanden werden, welche parallel zu einer größten Seitenfläche eines kleinsten geometrischen Quaders ist, welcher das Objekt gerade noch vollständig umschließt, und insbesondere durch den Mittelpunkt des Quaders verläuft. Unter einer "Längsrichtung" eines Elements soll insbesondere eine Richtung verstanden werden, die parallel zu einer längsten Seite eines kleinsten gedachten, das Element gerade noch umschließenden Quaders ausgerichtet ist. Unter einer "Querrichtung" eines Elements soll insbesondere eine Richtung verstanden werden, die parallel zu einer zweitlängsten Seite eines kleinsten gedachten, das Element gerade noch umschließenden Quaders ausgerichtet ist. Dadurch kann insbesondere ein schneller Montagevorgang erreicht werden. Zudem kann vorteilhaft eine hohe Flexibilität erreicht werden, wobei Toleranzen vorteilhaft kompensiert werden können.

Zudem wird vorgeschlagen, dass der zumindest eine zweite Steckverbinder zumindest ein zweites Führungselement aufweist, das dazu vorgesehen ist, eine Herstellung einer Verbindung zwischen dem zumindest einen ersten Steckverbinder und dem zumindest einen zweiten Steckverbinder insbesondere in der Montagerichtung zu erleichtern. Das zumindest eine zweite Führungselement und das zumindest eine erste Führungselement sind insbesondere korrespondierend zueinander ausgebildet und wirken bei einer Herstellung einer Verbindung zwischen dem zumindest einen ersten Steckverbinder und dem zumindest einen zweiten Steckverbinder zusammen. Dadurch kann insbesondere eine geringe Montagezeit und/oder eine preiswerte Ausgestaltung erreicht werden.

Es sind verschiedene, einem Fachmann als sinnvoll erscheinende Ausgestaltungen des zumindest einen zweiten Steckverbinders denkbar. Beispielsweise könnte ein Abstand zwischen den beiden zweiten Kontaktelementen wenigstens im Wesentlichen konstant ausgebildet sein, wobei der zumindest eine zweite Steckverbinder insbesondere einen wenigstens im Wesentlichen starren zweiten Verbindungsbereich aufweisen könnte. In einer alternativen Ausgestaltung des zumindest einen zweiten Steckverbinders weist der zumindest eine zweite Steckverbinder jedoch zumindest einen elastischen zweiten Verbindungsbereich auf, der dazu vorgesehen ist, einen Abstand zwischen den beiden zweiten Kontaktelementen, insbesondere einen Abstand zwischen Haupterstreckungsebenen der beiden zweiten Kontaktelemente, zu verändern. Der zumindest eine zweite Steckverbinder ist wenigstens in dem zumindest einen elastischen zweiten Verbindungsbereich aus wenigstens einem elastischen Material, beispielsweise aus elastischem Kunststoff und/oder aus leicht beweglichem Metall, ausgebildet und/oder derart geformt, dass eine Formveränderlichkeit gegeben ist. Dadurch kann insbesondere eine hohe Flexibilität erreicht werden, wobei Toleranzen vorteilhaft kompensiert werden können.

Es sind verschiedene, einem Fachmann als sinnvoll erscheinende Anzahlen an Paaren von zweiten Kontaktelementen des zumindest einen zweiten Steckverbinders denkbar. Beispielsweise könnte der zumindest eine zweite Steckverbinder insbesondere ausschließlich das zumindest eine Paar von zweiten Kontaktelementen aufweisen. Alternativ weist der zumindest eine zweite Steckverbinder jedoch zumindest ein weiteres Paar von zweiten Kontaktelementen auf, das insbesondere zu dem zumindest einen Paar von zweiten Kontaktelementen benachbart angeordnet und vorteilhaft mit dem zumindest einen Paar von zweiten Kontaktelementen durch einen insbesondere elastischen und/oder starren zweiten Verbindungsbereich verbunden ist. Darüber hinaus könnte der zumindest eine zweite Steckverbinder eine größere Anzahl an weiteren Paaren von zweiten Kontaktelementen aufweisen, die insbesondere benachbart zueinander angeordnet und vorteilhaft miteinander durch einen insbesondere elastischen und/oder starren zweiten Verbindungsbereich miteinander verbunden sein könnten. Entsprechendes gilt für den zumindest einen ersten Steckverbinder. Dadurch kann insbesondere eine flexible Ausgestaltung erreicht werden. Darüber hinaus können insbesondere hohe Stromstärken, insbesondere von mindestens 50 A, vorteilhaft von mindestens 60 A und besonders vorteilhaft von mindestens 70 A geführt werden.

Weitere Vorteile ergeben sich aus der folgenden Zeichnungsbeschreibung. In der Zeichnung sind Ausführungsbeispiele der Erfindung dargestellt. Die Zeichnung, die Beschreibung und die Ansprüche enthalten zahlreiche Merkmale in Kombination. Der Fachmann wird die Merkmale zweckmäßigerweise auch einzeln betrachten und zu sinnvollen weiteren Kombinationen zusammenfassen.

Es zeigen:
- Fig. 1: ein Hausgerät mit einer Hausgerätevorrichtung in einer schematischen Draufsicht,
- Fig. 2: einen zweiten Steckverbinder der Hausgerätevorrichtung in einer schematischen, perspektivischen Darstellung,
- Fig. 3: einen ersten Steckverbinder und den zweiten Steckverbinder der Hausgerätevorrichtung in einem ersten Montageschritt in einer schematischen Darstellung,
- Fig. 4: den ersten Steckverbinder und den zweiten Steckverbinder der Hausgerätevorrichtung in einem weiteren Montageschritt in einer schematischen perspektivischen Darstellung,
- Fig. 5: den ersten Steckverbinder und den zweiten Steckverbinder der Hausgerätevorrichtung in einem weiteren Montageschritt in einer schematischen Darstellung,
- Fig. 6: den ersten Steckverbinder und den zweiten Steckverbinder der Hausgerätevorrichtung in einem weiteren Montageschritt in einer schematischen perspektivischen Darstellung,
- Fig. 7: eine Leiterplatte, den ersten Steckverbinder und den zweiten Steckverbinder der Hausgerätevorrichtung in einem montierten Zustand in einer schematischen Darstellung von oben,
- Fig. 8: die Leiterplatte und erste Eingriffselemente des ersten Steckverbinders in dem montierten Zustand in einer schematischen Darstellung von unten,
- Fig. 9: einen alternativen zweiten Steckverbinder und den ersten Steckverbinder der Hausgerätevorrichtung in einer schematischen Darstellung und
- Fig. 10: einen alternativen zweiten Steckverbinder und zwei erste Steckverbinder der Hausgerätevorrichtung in einer schematischen perspektivischen Darstellung.

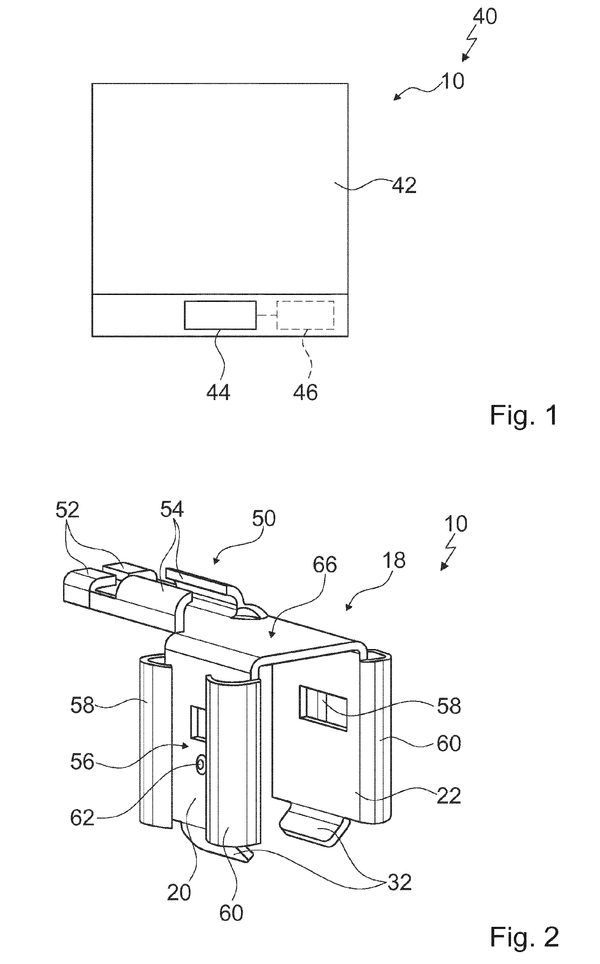

Fig. 1 zeigt ein Hausgerät 40, das als Kochfeld ausgebildet ist, mit einer Hausgerätevorrichtung 10, die als Kochfeldvorrichtung ausgebildet ist. Das Hausgerät 40 umfasst eine Kochfeldplatte 42 zu einem Aufstellen von Gargeschirr. Zudem umfasst das Hausgerät 40 mehrere Heizelemente (nicht dargestellt), die jeweils dazu vorgesehen sind, auf der Kochfeldplatte 42 oberhalb der Heizelemente aufgestelltes Gargeschirr zu erhitzen. Das Hausgerät 40 umfasst eine Bedieneinheit 44 zu einer Eingabe und/oder Auswahl von Betriebsparametern, beispielsweise einer Heizleistung und/oder einer Heizleistungsdichte und/oder einer Heizzone. Die Bedieneinheit 44 ist zu einer Ausgabe eines Werts eines Betriebsparameters an einen Bediener vorgesehen.

Die Hausgerätevorrichtung 10 umfasst eine Steuereinheit 46, die dazu vorgesehen ist, in Abhängigkeit von mittels der Bedieneinheit 44 eingegebener Betriebsparameter Aktionen auszuführen und/oder Einstellungen zu verändern. Die Steuereinheit 46 regelt in einem Heizbetriebszustand eine Energiezufuhr zu den Heizelementen. Hierzu steuert die Steuereinheit 46 eine Leistungsversorgung der Hausgerätevorrichtung 10 an, um die aktivierten Heizelemente mit elektrischem Strom zu versorgen. Die Hausgerätevorrichtung 10 umfasst mindestens eine elektrische Leitung (nicht dargestellt), die zu einem Transport von elektrischem Strom vorgesehen ist. Die elektrische Leitung könnte beispielsweise durch ein Kabel, einen Draht und/oder einen Litzendraht gebildet sein.

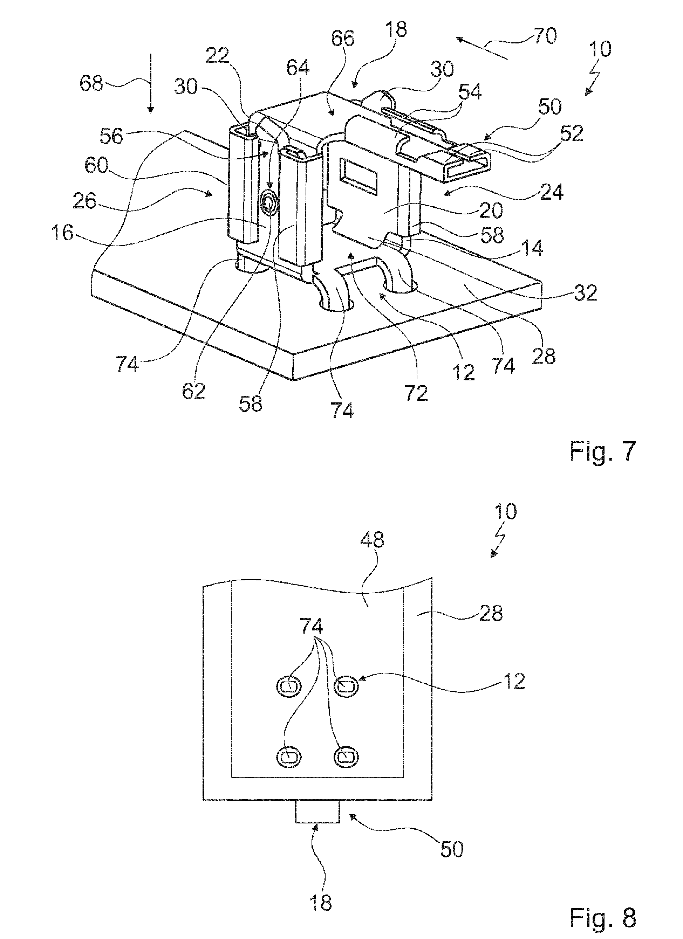

Zudem umfasst die Hausgerätevorrichtung 10 zu einem Transport von elektrischem Strom eine Leiterplatte 28 (vgl. Fig. 7 und 8). Die Leiterplatte 28 weist eine Leiterbahn 48 auf, die in einem Betriebszustand zu einem Transport von elektrischem Strom vorgesehen ist. Die Leiterbahn 48 ist einstückig mit der Leiterplatte 28 ausgebildet und verläuft auf einer Oberfläche der Leiterplatte 28. Zu einer Verbindung zwischen der Leiterbahn 48 der Leiterplatte 28 und der elektrischen Leitung umfasst die Hausgerätevorrichtung 10 einen ersten Steckverbinder 12 und einen zweiten Steckverbinder 18 (vgl. Fig. 2 bis 6), die zu einer Herstellung einer elektrisch leitenden Verbindung 24, 26 zusammenwirken. Der erste Steckverbinder 12 weist ein Paar von ersten Kontaktelementen 14, 16 auf. Analog zu dem Paar an ersten Kontaktelementen 14, 16 des ersten Steckverbinders 12 weist der zweite Steckverbinder 18 ein Paar von zweiten Kontaktelementen 20, 22 auf.

Der zweite Steckverbinder 18 weist einen zweiten Anschlussbereich 50 auf, der zu einer Kontaktierung mit der elektrischen Leitung vorgesehen ist (vgl. Fig. 2). In dem zweiten Anschlussbereich 50 weist der zweite Steckverbinder 18 vier zweite Fixierelemente 52, 54 auf, mittels welchen die elektrische Leitung in einem montierten Zustand an dem zweiten Steckverbinder 18 festgeklemmt ist. Die zweiten Fixierelemente 52, 54 sind durch Laschen gebildet. Jeweils zwei der zweiten Fixierelemente 52, 54 bilden ein Paar von zweiten Fixierelementen 52, 54 aus. Die beiden zweiten Fixierelemente 52, 54 eines Paars von zweiten Fixierelementen 52, 54 weisen jeweils aufeinander zu. Die Paare von zweiten Fixierelementen 52, 54 sind unterschiedlicher Größe.

Die Paare von zweiten Fixierelementen 52, 54 und das Paar von zweiten Kontaktelementen 20, 22 sind miteinander verbunden. Der zweite Steckverbinder 18 weist einen zweiten Verbindungsbereich 66 auf, der die Paare von zweiten Fixierelementen 52, 54 und das Paar von zweiten Kontaktelementen 20, 22 miteinander verbindet. In dem zweiten Verbindungsbereich 66 ist der zweite Steckverbinder 18 im Wesentlichen starr ausgebildet. Hierdurch kann vorteilhaft eine Montagehilfe bereitgestellt werden, durch welche ein Monteur in einfacher Weise, beispielsweise mit seinem Daumen, eine Verbindung zwischen dem ersten Steckverbinder 12, der beispielsweise auf der Leiterplatte 28 befestigt sein kann, und dem zweiten Steckverbinder 18 herstellen kann.

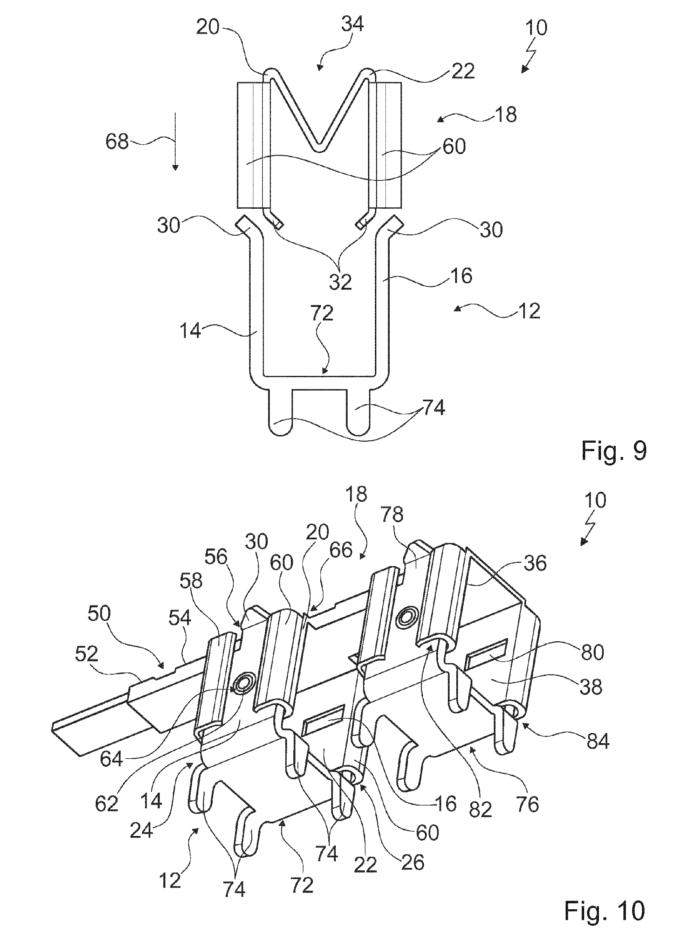

In einer alternativen Ausgestaltung weist der zweite Steckverbinder 18 einen elastischen zweiten Verbindungsbereich 34 auf, der dazu vorgesehen ist, einen Abstand zwischen den beiden zweiten Kontaktelementen 20, 22 zu verändern (vgl. Fig. 9). Bei Betrachtung in einer Frontansicht weist der zweite Steckverbinder 18 eine im Wesentlichen M-förmige Gestalt auf. Demgegenüber weist der zweite Steckverbinder 18 im vorliegenden Ausführungsbeispiel bei Betrachtung in einer Frontansicht eine im Wesentlichen U-förmige Gestalt auf.

In dem montierten Zustand bildet das Paar von zweiten Kontaktelementen 20, 22 gemeinsam mit dem Paar von ersten Kontaktelementen 14, 16 zwei zueinander parallele Verbindungen 24, 26 aus (vgl. Fig. 7). Zu einer Herstellung der beiden zueinander parallelen Verbindungen 24, 26 werden die ersten Kontaktelemente 14, 16 in die zweiten Kontaktelemente 20, 22 eingeführt, insbesondere eingesteckt. Die zweiten Kontaktelemente 20, 22 des zumindest einen Paars von zweiten Kontaktelementen 20, 22 weisen jeweils einen Kabelschuh auf. Jedes zweite Kontaktelement 20, 22 weist einen Aufnahmebereich 56 auf, der zu einer Aufnahme eines der beiden ersten Kontaktelemente 14, 16 vorgesehen ist. Die zweiten Kontaktelemente 20, 22 weisen jeweils zwei zweite Klemmelemente 58, 60 auf, die in dem montierten Zustand die ersten Kontaktelemente 14, 16 in dem Aufnahmebereich 56 befestigen. Die zweiten Klemmelemente 58, 60 sind jeweils als Laschen ausgebildet.

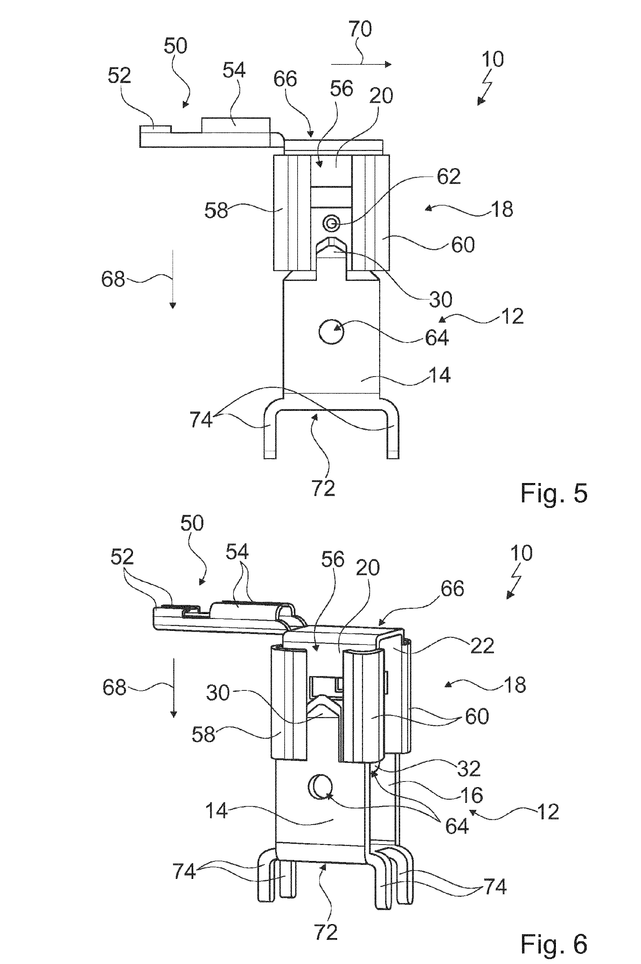

Die zweiten Kontaktelemente 20, 22 weisen zudem jeweils ein zweites Sicherungselement 62 auf, das in dem montierten Zustand eine Verbindung zwischen dem ersten Steckverbinder 12 und dem zweiten Steckverbinder 18 sichert (vgl. Fig. 5). Die zweiten Sicherungselemente 62 sind als insbesondere halbkugelförmige Vorsprünge ausgebildet, welche in dem Aufnahmebereich 56 angeordnet sind. Analog zu den zweiten Sicherungselementen 62 der zweiten Kontaktelemente 20, 22 weisen die ersten Kontaktelemente 14, 16 jeweils ein erstes Sicherungselement 64 auf, das in dem montierten Zustand mit einem der zweiten Sicherungselemente 62 zusammenwirkt. Die ersten Sicherungselemente 64 sind als insbesondere kreisförmige Ausnehmungen ausgebildet und zu einer Aufnahme der zweiten Sicherungselemente 62 vorgesehen.

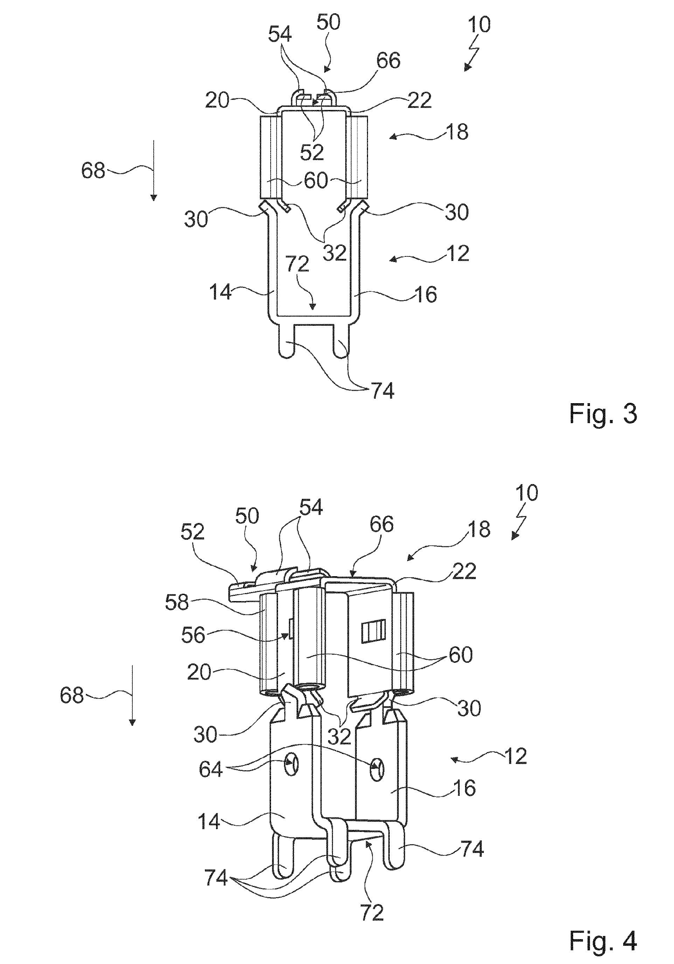

Für jedes Paar von zweiten Kontaktelementen 20, 22 weist der zweite Steckverbinder 18 ein Paar von zweiten Führungselementen 32 auf, die eine Herstellung einer Verbindung zwischen dem ersten Steckverbinder 12 und dem zweiten Steckverbinder 18 erleichtern. Bei einer Montage erleichtern die zweiten Führungselemente 32 die Herstellung der Verbindung zwischen dem ersten Steckverbinder 12 und dem zweiten Steckverbinder 18 in einer Montagerichtung 68 (vgl. Fig. 3 bis 6). Die zweiten Führungselemente 32 des zweiten Steckverbinders 18 sind an einem dem zweiten Verbindungsbereich 66 abgewandten Ende der zweiten Kontaktelemente 20, 22 angeordnet. Jedes zweite Führungselement 32 ist schräg zu einer Haupterstreckungsebene ausgerichtet, die von einer Oberfläche des entsprechenden zweiten Kontaktelements 20, 22 gebildet ist. Hierbei schließt jedes zweite Führungselement 32 mit der Haupterstreckungsebene einen Winkel in einem Bereich zwischen 30° und 60°, vorzugsweise von im Wesentlichen 45° ein. Die zweiten Führungselemente 32 sind einander zugewandt an den zweiten Kontaktelementen 20, 22 angeordnet. Die zweiten Führungselemente 32 bilden Gleitflächen aus, welche bei einem Herstellen einer Verbindung zwischen dem ersten Steckverbinder 12 und dem zweiten Steckverbinder 18 mit korrespondierenden ersten Führungselementen 30 des ersten Steckverbinders 12 zusammenwirken.

Analog zu den zweiten Führungselementen 32 des zweiten Steckverbinders 18 weist der erste Steckverbinder 12 für jedes Paar von ersten Kontaktelementen 14, 16 ein Paar an ersten Führungselementen 30 auf, die eine Herstellung einer Verbindung zwischen dem zumindest einen ersten Steckverbinder 12 und dem zumindest einen zweiten Steckverbinder 18 erleichtern. Der erste Steckverbinder 12 weist einen ersten Verbindungsbereich 72 auf, der die beiden ersten Kontaktelemente 14, 16 miteinander verbindet. Die ersten Führungselemente 30 sind an den ersten Kontaktelementen 14, 16 angeordnet. Hierbei sind die ersten Führungselemente 30 an einem dem ersten Verbindungsbereich 72 abgewandten Ende der ersten Kontaktelemente 14, 16 angeordnet. Jedes erste Führungselement 30 ist schräg zu einer Haupterstreckungsebene ausgerichtet, die von einer Oberfläche des entsprechenden ersten Kontaktelements 14, 16 gebildet ist. Hierbei schließt jedes erste Führungselement 30 mit der Haupterstreckungsebene einen Winkel in einem Bereich zwischen 30° und 60°, vorzugsweise von im Wesentlichen 45° ein. Die ersten Führungselemente 30 sind einander abgewandt an den ersten Kontaktelementen 14, 16 angeordnet. Die ersten Führungselemente 30 bilden Gleitflächen aus, welche bei einem Herstellen einer Verbindung zwischen dem ersten Steckverbinder 12 und dem zweiten Steckverbinder 18 mit den zweiten Führungselementen 32 des zweiten Steckverbinders 18 zusammenwirken.

Die ersten Führungselemente 30 erleichtern das Herstellen der Verbindung zwischen dem ersten Steckverbinder 12 und dem zweiten Steckverbinder 18 in der Montagerichtung 68. Darüber hinaus erleichtern die ersten Führungselemente 30 das Herstellen der Verbindung zwischen dem ersten Steckverbinder 12 und dem zweiten Steckverbinder 18 in einer Querrichtung 70. Die ersten Führungselemente 30 weisen bei Betrachtung in einer Frontansicht eine dreieckige Gestalt auf. Sollten die ersten Führungselemente 30 bei dem Herstellen der Verbindung zwischen dem ersten Steckverbinder 12 und dem zweiten Steckverbinder 18 an einem der zweiten Klemmelemente 58, 60 anstoßen, so leiten die ersten Führungselemente 30 die ersten Kontaktelemente 14, 16 bei Bewegung in der Montagerichtung 68 automatisch in den Aufnahmebereich 56 der zweiten Kontaktelemente 20, 22.

Der erste Steckverbinder 12 ist zu einem Kontakt mit der Leiterplatte 28 vorgesehen. In dem montierten Zustand ist der erste Steckverbinder 12 mit der Leiterplatte 28 verbunden (vgl. Fig. 7). Der erste Verbindungsbereich 72 ist in dem montierten Zustand in einem Nahbereich der Leiterplatte 28 angeordnet. Die ersten Kontaktelemente 14, 16 sind von der Leiterplatte 28 abweisend an dem ersten Verbindungsbereich 72 angeordnet und im Wesentlichen senkrecht zu dem ersten Verbindungsbereich 72 ausgerichtet. Zudem sind die ersten Kontaktelemente 14, 16 im Wesentlichen senkrecht zu der Leiterplatte 28 ausgerichtet. Der erste Steckverbinder 12 weist eine im Wesentlichen U-förmige Gestalt auf.

Zu einer Herstellung einer Verbindung zwischen dem ersten Steckverbinder 12 und der Leiterplatte 28 weist der erste Steckverbinder 12 vier erste Eingriffselemente 74 auf (vgl. Fig. 3 bis 8). In einem Übergangsbereich zwischen dem ersten Verbindungsbereich 72 und einem der ersten Kontaktelemente 14, 16 sind jeweils zwei der ersten Eingriffselemente 74 angeordnet. Im Folgenden wird lediglich eines der ersten Eingriffselemente 74 beschrieben. Das erste Eingriffselement 74 greift in dem montierten Zustand durch die Leiterplatte 28 hindurch. Die Leiterplatte 28 weist vier Ausnehmungen auf, die zu einer Aufnahme jeweils eines der ersten Eingriffselemente 74 vorgesehen sind. Im Folgenden wird lediglich eine der Ausnehmungen beschrieben. Die Ausnehmung ist in einem Bereich der Leiterbahn 48 angeordnet. Ein Querschnitt der Ausnehmung ist größer als ein Querschnitt des ersten Eingriffselements 74, um insbesondere mögliche Toleranzen ausgleichen zu können. Das erste Eingriffselement 74 weist in eine den ersten Kontaktelementen 14, 16 abgewandte und der Leiterplatte 28 zugewandte Richtung. Hierbei ist das erste Eingriffselement 74 im Wesentlichen parallel zu der Montagerichtung 68 ausgerichtet. Die Montagerichtung 68 ist im Wesentlichen senkrecht zu einer Haupterstreckungsebene der Leiterplatte 28 ausgerichtet.

Das erste Eingriffselement 74 ist stiftförmig ausgebildet. Eine Erstreckung des ersten Eingriffselements 74 ist größer als eine Erstreckung, insbesondere eine Dicke, der Leiterplatte 28 in der Montagerichtung 68. In dem montierten Zustand ragt das erste Eingriffselement 74 durch die Leiterplatte 28 hindurch (vgl. Fig. 8). Das erste Eingriffselement 74 ist in dem montierten Zustand elektrisch leitend mit der Leiterbahn 48 der Leiterplatte 28 verbunden. Im vorliegenden Ausführungsbeispiel ist das Eingriffselement 74 an die Leiterplatte 28, und zwar an die Unterseite der Leiterplatte 28, angelötet.

In einem Verfahren zu einer Montage der Hausgerätevorrichtung 10 könnten beispielsweise der erste Steckverbinder 12 und der zweite Steckverbinder 18 miteinander verbunden werden, wobei anschließend der erste Steckverbinder 12 mit der Leiterplatte 28 verbunden werden könnte. Im vorliegenden Ausführungsbeispiel wird zunächst der erste Steckverbinder 12 mit der Leiterplatte 28 verbunden (vgl. Fig. 7 und 8). Hierbei wird der erste Steckverbinder 12 in der Montagerichtung 68 der Leiterplatte 28 angenähert. Bei der Annäherung greift das erste Eingriffselement 74 durch die Ausnehmung der Leiterplatte 28 hindurch. Ist das Eingriffselement 74 in der Ausnehmung der Leiterplatte 28 angeordnet, wird das Eingriffselement 74 an der Unterseite der Leiterplatte 28 in elektrisch leitendem Kontakt mit der Leiterbahn 48 verbunden. Anschließend wird der zweite Steckverbinder 18 in der Montagerichtung 68 dem ersten Steckverbinder 12 angenähert. Bei Bewegung in der Montagerichtung 68 wirken die ersten Führungselemente 30 und die zweiten Führungselemente 32 zusammen und erleichtern die Herstellung der Verbindung zwischen dem ersten Steckverbinder 12 und dem zweiten Steckverbinder 18.

Die ersten Führungselemente 30 erleichtern eine Herstellung eines Eingriffs zwischen dem ersten Sicherungselement 64 und dem zweiten Sicherungselement 62. Hierzu wird der zweite Steckverbinder 18 in die Montagerichtung 68 gedrückt, beispielsweise indem eine Kraft auf den zweiten Verbindungsbereich 66 ausgeübt wird. In dem montierten Zustand, in welchem die Sicherungselemente 62, 64 miteinander in Eingriff sind, bilden das Paar von ersten Kontaktelementen 14, 16 und das Paar von zweiten Kontaktelementen 20, 22 die beiden zueinander parallele Verbindungen 24, 26 aus. Die beiden zueinander parallelen Verbindungen 24, 26 sind einander gleichwertig. In einem Betriebszustand führen die beiden zueinander parallelen Verbindungen 24, 26 im Wesentlichen gleiche Stromstärken. Im vorliegenden Ausführungsbeispiel führt jede der beiden zueinander parallelen Verbindungen 24, 26 eine Stromstärke von im Wesentlichen 20 A. Durch eine Kontaktfläche zwischen einem ersten Kontaktelement 14, 16 und einem zweiten Kontaktelement 20, 22 fließt ein Strom mit einer Stromstärke von im Wesentlichen 20 A. Das Eingriffselement 74 führt einen elektrischen Strom mit einer Stromstärke von im Wesentlichen 10 A.

Die stromführenden Bereiche der Eingriffselemente 74 sind im Wesentlichen gleich groß wie die stromführenden Bereiche der ersten Kontaktelemente 14, 16 sowie der zweiten Kontaktelemente 20, 22. Die stromführenden Bereiche der ersten Kontaktelemente 14, 16 sowie der zweiten Kontaktelemente 20, 22 sind im vorliegenden Ausführungsbeispiel durch eine Vorderkante der zweiten Klemmelemente 58, 60 gebildet. In einer alternativen Ausgestaltung ist denkbar, dass die Kontaktflächen zwischen den ersten Kontaktelementen und den zweiten Kontaktelementen die stromführenden Bereiche der ersten Kontaktelemente sowie der zweiten Kontaktelemente ausbilden.

In dem Betriebszustand führen der erste Steckverbinder 12 und der zweite Steckverbinder 18 eine Gesamtstromstärke von im Wesentlichen 40 A. Sollte eine höhere Gesamtstromstärke erforderlich sein, so ist eine Ausgestaltung des zweiten Steckverbinders 18 wie in Fig. 9 dargestellt denkbar. Die Hausgerätevorrichtung 10 weist den ersten Steckverbinder 12 und einen weiteren ersten Steckverbinder 76 auf, der ein weiteres Paar von ersten Kontaktelementen 78, 80 aufweist. Der weitere erste Steckverbinder 76 ist analog zu dem ersten Steckverbinder 12 ausgebildet. Der zweite Steckverbinder 18 weist neben dem Paar von zweiten Kontaktelementen 20, 22 ein weiteres Paar von zweiten Kontaktelementen 36, 38 auf. Das weitere Paar von zweiten Kontaktelementen 36, 38 ist analog zu dem Paar von zweiten Kontaktelementen 20, 22 ausgebildet. In dem montierten Zustand bilden das weitere Paar von zweiten Kontaktelementen 36, 38 und das weitere Paar von ersten Kontaktelementen 78, 80 des weiteren ersten Steckverbinders 76 zwei zueinander parallele Verbindungen 82, 84 aus. In dem Betriebszustand führen die zueinander parallelen Verbindungen 24, 26, 82, 84 im Wesentlichen gleiche Stromstärken. Eine zu einem Lösen der Verbindung zwischen den ersten Steckverbindern 12, 76 und dem zweiten Steckverbinder 18 benötigte Kraft erhöht sich insbesondere um einen Faktor, der durch eine Anzahl an weiteren Paaren von zweiten Kontaktelementen 36, 38 festgelegt ist.

Der erste Steckverbinder 12 ist als einstückiges Bauteil ausgebildet. Hierbei sind die ersten Kontaktelemente 14, 16, der erste Verbindungsbereich 72, die ersten Führungselemente 30 und das erste Eingriffselement 74 einstückig miteinander verbunden. Die zweiten Kontaktelemente 20, 22, der zweite Verbindungsbereich 34, 66, der zweite Anschlussbereich 50 und die zweiten Führungselemente 32 sind einstückig miteinander verbunden. Der zweite Steckverbinder 18 ist als einstückiges Bauteil ausgebildet. Hierbei könnte "einstückig" insbesondere zumindest stoffschlüssig verbunden bedeuten, wie beispielsweise durch einen Schweißprozess, einen Klebeprozess, einen Anspritzprozess und/oder einen anderen, dem Fachmann als sinnvoll erscheinenden Prozess. Vorzugsweise bedeutet "einstückig" in diesem Zusammenhang jedoch in einem Stück geformt, wie beispielsweise durch eine Herstellung aus einem Guss und/oder durch eine Herstellung in einem Ein- oder Mehrkomponentenspritzverfahren und vorteilhaft aus einem einzelnen Rohling.

In einer weiteren Ausgestaltung ist beispielsweise eine andere, insbesondere höhere Anzahl an ersten Steckverbindern und/oder zweiten Steckverbindern denkbar. Beispielsweise könnte für jedes Heizelement ein zweiter Steckverbinder vorgesehen sein, wobei für jedes Paar an zweiten Kontaktelementen jedes zweiten Steckverbinders ein erster Steckverbinder vorgesehen sein könnte.

Alternativ zu einer Ausgestaltung mit der insbesondere einzigen Leiterplatte ist denkbar, dass die Hausgerätevorrichtung eine größere Anzahl an Leiterplatten aufweist, beispielsweise zwei, drei, vier oder mehr Leiterplatten. Ebenfalls denkbar ist, dass eine Anzahl an Leiterplatten einer Anzahl an Heizelementen entspricht. Die Leiterplatte könnte alternativ zu der insbesondere einzigen Leiterbahn eine höhere Anzahl an Leiterbahnen aufweisen. Eine Anzahl an elektrischen Leitungen könnte beispielsweise einer gesamten Anzahl an Leiterbahnen entsprechen, wobei insbesondere die eine insbesondere einzige Leiterplatte die gesamte Anzahl an Leiterbahnen aufweisen könnte. Ebenfalls möglich ist, dass mehrere Leiterplatten vorgesehen sind, die gemeinsam die gesamte Anzahl an Leiterbahnen aufweisen.

### Bezugszeichen

- 10: Hausgerätevorrichtung
- 12: Erster Steckverbinder
- 14: Erstes Kontaktelement
- 16: Erstes Kontaktelement
- 18: Zweiter Steckverbinder
- 20: Zweites Kontaktelement
- 22: Zweites Kontaktelement
- 24: Erste Verbindung
- 26: Zweite Verbindung
- 28: Leiterplatte
- 30: Erstes Führungselement
- 32: Zweites Führungselement
- 34: Zweiter Verbindungsbereich
- 36: Zweites Kontaktelement
- 38: Zweites Kontaktelement
- 40: Hausgerät
- 42: Kochfeldplatte
- 44: Bedieneinheit
- 46: Steuereinheit
- 48: Leiterbahn
- 50: Zweiter Anschlussbereich
- 52: Zweites Fixierelement
- 54: Zweites Fixierelement
- 56: Aufnahmebereich
- 58: Zweites Klemmelement
- 60: Zweites Klemmelement
- 62: Zweites Sicherungselement
- 64: Erstes Sicherungselement
- 66: Zweiter Verbindungsbereich
- 68: Montagerichtung
- 70: Querrichtung
- 72: Erster Verbindungsbereich
- 74: Erstes Eingriffselement
- 76: Weiterer erster Steckverbinder
- 78: Erstes Kontaktelement
- 80: Erstes Kontaktelement
- 82: Weitere erste Verbindung
- 84: Weitere zweite Verbindung

## Patentansprüche

1. Hausgerätevorrichtung, insbesondere Kochfeldvorrichtung, mit zumindest einem ersten Steckverbinder (12), der zumindest ein Paar von ersten Kontaktelementen (14, 16) aufweist, und mit zumindest einem zweiten Steckverbinder (18), der zumindest ein Paar von zweiten Kontaktelementen (20, 22) aufweist, das in wenigstens einem montierten Zustand gemeinsam mit dem zumindest einen Paar von ersten Kontaktelementen (14, 16) zwei zueinander parallele Verbindungen (24, 26) ausbildet, wobei zumindest eines der zweiten Kontaktelemente (20, 22) einen Kabelschuh aufweist.

2. Hausgerätevorrichtung nach Anspruch 1, **dadurch gekennzeichnet, dass** die zweiten Kontaktelemente (20, 22) des zumindest einen Paars von zweiten Kontaktelementen (20, 22) jeweils einen Kabelschuh aufweisen.

3. Hausgerätevorrichtung nach Anspruch 1 oder 2, **dadurch gekennzeichnet, dass** die beiden zueinander parallelen Verbindungen (24, 26) in wenigstens einem Betriebszustand wenigstens im Wesentlichen gleiche Stromstärken führen.

4. Hausgerätevorrichtung nach einem der vorhergehenden Ansprüche, **gekennzeichnet durch** zumindest eine Leiterplatte (28), mit welcher der zumindest eine erste Steckverbinder (12) in dem wenigstens einen montierten Zustand verbunden ist.

5. Hausgerätevorrichtung nach einem der vorhergehenden Ansprüche, **dadurch gekennzeichnet, dass** der zumindest eine erste Steckverbinder (12) zumindest ein erstes Führungselement (30) aufweist, das dazu vorgesehen ist, eine Herstellung einer Verbindung zwischen dem zumindest einen ersten Steckverbinder (12) und dem zumindest einen zweiten Steckverbinder (18) zu erleichtern.

6. Hausgerätevorrichtung nach einem der vorhergehenden Ansprüche, **dadurch gekennzeichnet, dass** der zumindest eine zweite Steckverbinder (18) zumindest ein zweites Führungselement (32) aufweist, das dazu vorgesehen ist, eine Herstellung einer Verbindung zwischen dem zumindest einen ersten Steckverbinder (12) und dem zumindest einen zweiten Steckverbinder (18) zu erleichtern.

7. Hausgerätevorrichtung nach einem der vorhergehenden Ansprüche, **dadurch gekennzeichnet, dass** der zumindest eine zweite Steckverbinder (18) zumindest einen elastischen zweiten Verbindungsbereich (34) aufweist, der dazu vorgesehen ist, einen Abstand zwischen den beiden zweiten Kontaktelementen (20, 22) zu verändern.

8. Hausgerätevorrichtung nach einem der vorhergehenden Ansprüche, **dadurch gekennzeichnet, dass** der zumindest eine zweite Steckverbinder (18) zumindest ein weiteres Paar von zweiten Kontaktelementen (36, 38) aufweist.

9. Erster Steckverbinder für eine Hausgerätevorrichtung (10) nach einem der vorhergehenden Ansprüche.

10. Zweiter Steckverbinder für eine Hausgerätevorrichtung (10) nach einem der Ansprüche 1 bis 8.

11. Hausgerät, insbesondere Kochfeld, mit zumindest einer Hausgerätevorrichtung (10) nach einem der Ansprüche 1 bis 8.
